# EUROPÄISCHE PATENTSCHRIFT

(11) **EP 1 696 870 B1**
(45) Veröffentlichungstag und Bekanntmachung des Hinweises auf die Patenterteilung: **21.09.2011**
(21) Anmeldenummer: 04803609.9
(22) Anmeldetag: 08.12.2004
(51) Int. Cl.: A61K 8/41, A61Q 5/06, A61K 8/49

(54) **Tönungsmittel in Tuben**
Tinting agent in tubes
Agent de teinture en tubes

(30) Priorität: 17.12.2003 DE 10359538
(43) Veröffentlichungstag der Anmeldung: 06.09.2006
(73) Patentinhaber: Henkel AG & Co. KGaA, 40589 Düsseldorf (DE)
(72) Erfinder: KLEEN, Astrid, 22763 Hamburg (DE); AKRAM, Mustafa, 22457 Hamburg (DE); BIETZ, Susanne, 25336 Elmshorn (DE); HOEPFNER, Stefan, 22525 Hamburg (DE); MANNECK, Hartmut, 23860 Klein Wesenberg (DE)
(86) Internationale Anmeldenummer: PCT/EP2004/013929
(87) Internationale Veröffentlichungsnummer: WO 2005/058259

(56) Entgegenhaltungen:
- WO-A-01/97756
- WO-A-03/089330
- DE-A1- 10 162 640
- DE-A1- 19 945 486
- GB-A- 1 289 712
- US-A- 4 184 844
- US-A1- 2003 106 167
- US-A1- 2003 150 069

## Beschreibung

Die vorliegende Erfindung betrifft in einer Zweikammertube konfektionierte Zweikomponentenmittel zur Tönung und/oder Färbung keratinischer Fasern, sowie ein Verfahren zur Tönung und/oder Färbung keratinischer Fasern mit Hilfe dieses Zweikomponentenmittels.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Färbemittel auf Basis von Oxidationsfarbstoffen führen zu brillanten und dauerhaften Farbtönen. Sie bedingen allerdings den Einsatz starker Oxidationsmittel wie beispielsweise Wasserstoffperoxid-Lösungen. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Färbemittel auf der Basis direktziehender Farbstoffe kommen ohne Oxidationsmittel aus und können bei pH-Werten im Bereich des Neutralpunktes formuliert werden, ergeben aber weniger dauerhafte Färbungen. Das Aufziehvermögen der Farbstoffmoleküle auf das Haar, sowie der Glanz der gefärbten Haaren können in vielen Fällen ebenfalls nicht voll befriedigen.

Nicht zuletzt durch die starke Beanspruchung der Haare durch derartige farbverändernde Behandlungen, aber auch durch Dauerwellen, die Reinigung der Haare mit Shampoos und durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an eine Behandlung mit einem Pflegemittel beispielsweise die Nass- und Trockenkämmbarkeit des Haares, der Halt, die Festigkeit und die Fülle des Haares optimiert sein oder die Haare vor erhöhtem Spliss geschützt sein.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splissrate verringert.

Die zur Verfügung stehenden Wirkstoffe wirken im allgemeinen bevorzugt an der Haaroberfläche. So sind Wirkstoffe bekannt, welche dem Haar Glanz, Halt, Fülle, bessere Nass- oder Trockenkämmbarkeiten verleihen oder dem Spliss vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflusst werden kann. Es wurden in letzter Zeit auch Wirkstoffe vorgeschlagen, die dieser Änderung der inneren Struktur der Fasern nachhaltig entgegenwirken können.

In jüngster Zeit wurden sogenannte Kombinationspräparate entwickelt, um den Aufwand der üblichen mehrstufigen Verfahren, insbesondere bei der direkten Anwendung durch Verbraucher, zu verringern. Diese Präparate enthalten neben den üblichen Komponenten zur Tönung und/oder Färbung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die bekannten Wirkstoffe weisen jedoch teilweise den Nachteil auf, dass sie in den Tönungsmitteln nicht stabil formuliert werden können.

WO-A-03/089330, US-A-4 184 844 und GB-A-1 289 712 offenbaren zweikomponentige Zubereitungen zum Färben und Pflegen der Haare in Verpackungen mit zwei Kammern.

Es wurde nunmehr überraschenderweise gefunden, dass stabile Tönungsmittel mit einer hervorragenden Pflegewirkung erhalten werden können, wenn ein Mittel enthaltend mindestens einen direktziehenden Farbstoff und/oder eine Vorstufe eines naturanalogen Farbstoffs und ein Mittel enthaltend mindestens eine Pflegekomponente getrennt voneinander in einer Zweikammertube konfektioniert werden.

Gegenstand der vorliegenden Erfindung sind daher Zweikomponentenmittel zur Tönung und/oder Färbung keratinischer Fasern, bestehend aus einer ersten Zubereitung (A), enthaltend mindestens einen direktziehenden Farbstoff und/oder mindestens eine Vorstufe eines naturanalogen Farbstoffs, und einer zweiten Zubereitung (B), enthaltend mindestens einen Pflegestoff, wobei die beiden Zubereitungen getrennt voneinander in den Kammern einer Zweikammertube konfektioniert sind.

Die erfindungsgemäßen Zweikomponentenmittel zeichnen sich durch eine hervorragende Pflege- und Färbeleistung und eine hohe Stabilität aus. Darüber hinaus ist gewährleistet, dass der Verbraucher die Komponenten im vom Hersteller geplanten Mischungsverhältnis appliziert. Auf diese Weise ist einerseits die Produktsicherheit erhöht und andererseits gewährleistet, dass das Produkt die gewünschte Leistung erbringt.

Die Zubereitung (A) der erfindungsgemäßen Zweikomponentenmittel enthält in einer ersten bevorzugten Ausführungsform mindestens einen direktziehenden Farbstoff. Die direktziehenden Farbstoffe sind vorzugsweise ausgewählt aus den Nitrophenylendiaminen, den Nitroaminophenolen, den Azofarbstoffen, den Anthrachinonen oder den Indophenolen. Besonders bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner kann es erfindungsgemäß bevorzugt sein, dass die Mittel mindestens einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die mindestens einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

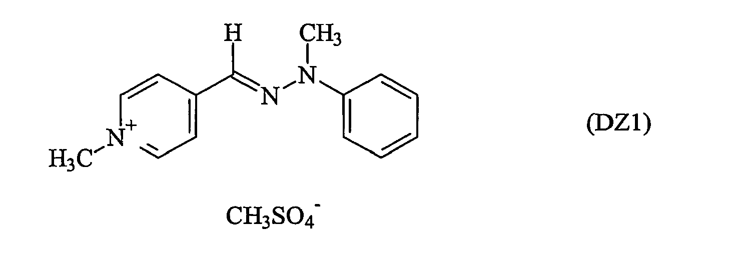

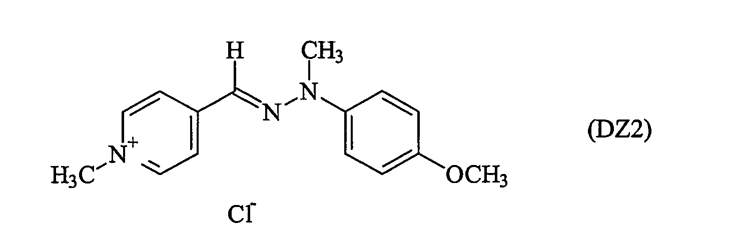

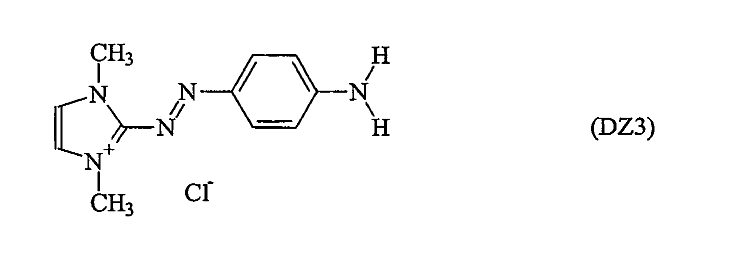

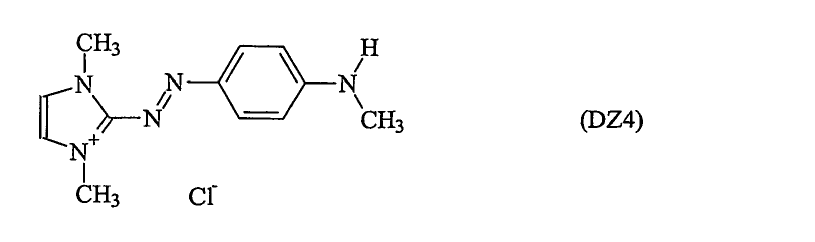

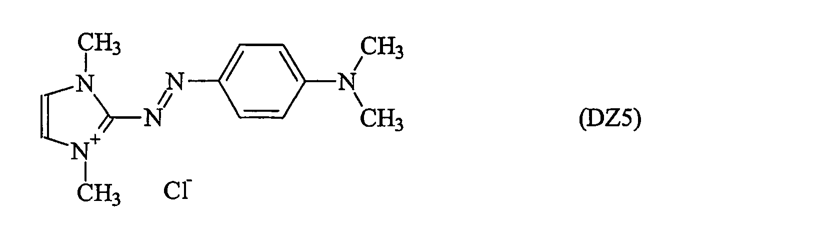

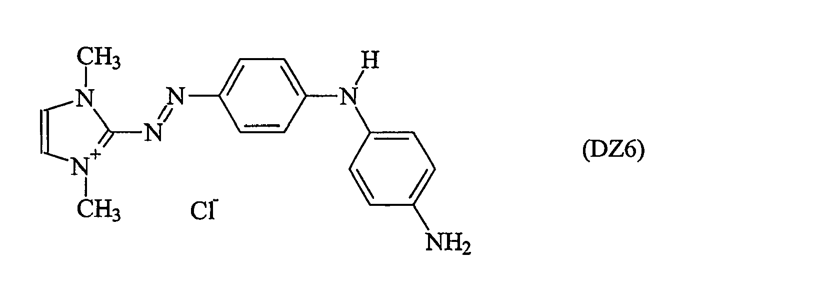

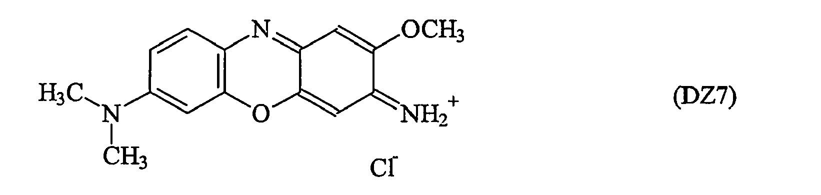

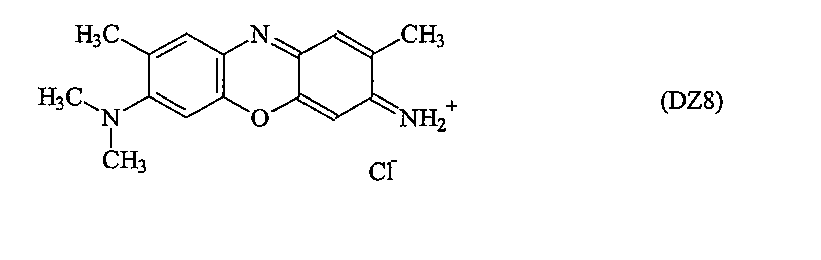

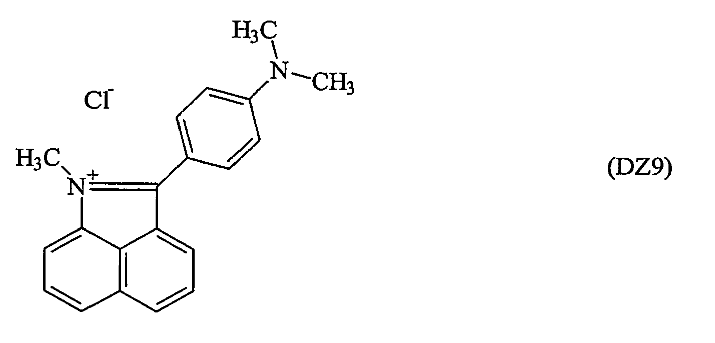

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Weiterhin können die erfindungsgemäßen Zubereitungen auch in der Natur vorkommende direktziehende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die erfindungsgemäßen Zweikomponentenmittel enthalten die direktziehenden Farbstoffe bevorzugt in einer Menge von 0,01 bis 20 Gew.-%, bezogen auf die gesamte Anwendungszubereitung, d.h. bezogen auf die Summe der Zubereitungen (A) und (B).

Die erfindungsgemäßen Zweikomponentenmittel enthalten in der Zubereitung (A) mindestens eine Vorstufe eines naturanalogen Farbstoffs.

Als Vorstufen naturanaloger Farbstoffe werden Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer bevorzugten Ausführungsform enthalten die Zweikomponentenmittel daher mindestens ein Indol- und/oder ein Indolinderivat.

Besonders gut als Vorstufen naturanaloger Haarfarbstoffe geeignet sind Derivate des 5,6-Dihydroxyindolins der Formel (la), 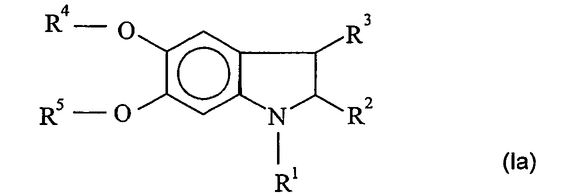 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
   sowie physiologisch verträgliche Salze dieser Verbindungen mit einer organischen oder anorganischen Säure.

Beispiele für die als Substituenten in den erfindungsgemäßen Verbindungen genannten C₁- bis C₄-Alkylgruppen sind die Gruppen Methyl, Ethyl, Propyl, Isopropyl und Butyl. Ethyl und Methyl sind bevorzugte Alkylgruppen. Weiterhin können als bevorzugte Beispiele für eine C₁- bis C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 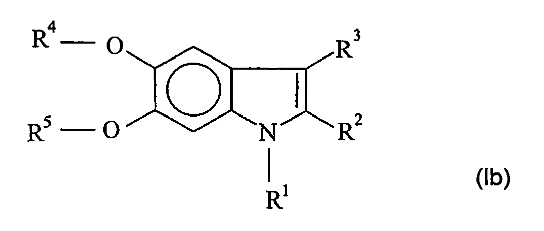 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Beispiele für die als Substituenten in den erfindungsgemäßen Verbindungen genannten C₁- bis C₄-Alkylgruppen sind die Gruppen Methyl, Ethyl, Propyl, Isopropyl und Butyl. Ethyl und Methyl sind bevorzugte Alkylgruppen. Weiterhin können als bevorzugte Beispiele für eine C₁- bis C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Mitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-%, jeweils bezogen auf die gesamte Anwendungszubereitung enthalten.

Es kann erfindungsgemäß bevorzugt sein, das Indolin- oder Indolderivat in den Zweikomponentenmitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin. Dabei spielt es erfindungsgemäß keine Rolle, ob die Aminosäure gemeinsam mit dem Indolin- oder Indolderivat in der Zubereitung (A) enthalten ist oder ob die Aminosäure getrennt von dem Indolin- oder Indolderivat in der Zubereitung (B) enthalten ist.

Die Zubereitung (B) des erfindungsgemäßen Zweikomponentenmittels enthält erfindungsgemäß mindestens einen Pflegestoff.

Im Rahmen einer ersten bevorzugten Ausführungsform enthält das erfindungsgemäße Zweikomponentenmittel als Pflegestoff mindestens ein kationisches Tensid.

Erfindungsgemäß bevorzugt sind kationische Tenside vom Typ der quartären Ammoniumverbindungen, der Esterquats und der Amidoamine. Bevorzugte quatäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Die kationischen Tenside sind in den erfindungsgemäßen Zweikomponentenmitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Im Rahmen einer zweiten bevorzugten Ausführungsform der vorliegenden Erfindung enthalten die Zweikomponentenmittel als Pflegestoff mindestens ein pflegendes Polymer.

Eine erste Gruppe der pflegenden Polymere sind die kationischen Polymere. Unter kationischen Polymeren sind erfindungsgemäß Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁-₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allqemeinen Formel (G1-I), 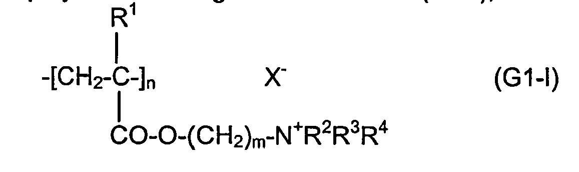 in der R¹= -H oder -CH₃ ist, R², R³ und R⁴ unabhängig voneinander ausgewählt sind aus C₁₋₄-Alkyl-, C₂₋₄-Alkenyl- oder C₁₋₄-Hydroxyalkylgruppen, m = 1, 2, 3 oder 4, n eine natürliche Zahl, vorzugsweise eine natürliche Zahl von 1 bis 5000, und X- ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxyprodispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quatemisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazollummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäß einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugt eingesetzte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäß einsetzbaren, kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Weitere erfindungsgemäß einsetzbare pflegende Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten amphoteren Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Zweikomponentenmittel enthalten die pflegenden Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Im Rahmen einer dritten bevorzugten Ausführungsform enthalten die erfindungsgemäßen Zweikomponentenmittel mindestens einen UV-Filter. Die erfindungsgemäß geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestem.

Beispiele für erfindungsgemäß verwendbare UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianifino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Aminobenzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.

Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, dass das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, dass der molare Extinktionskoeffizient des UV-Filters am Absorptionsmaximum oberhalb von 15.000, insbesondere oberhalb von 20.000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so dass der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)_{X}-N⁺R¹R²R³ X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X⁻ für ein physiologisch verträgliches Anion. Im Rahmen dieser allgemeinen Struktur steht x bevorzugt für die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter sind in den erfindungsgemäßen Mitteln üblicherweise in Mengen 0,01-5 Gew.-%, bezogen auf die gesamte Anwendungszubereitung, enthalten. Mengen von 0,1-2,5 Gew.-% sind bevorzugt.

Im Rahmen einer vierten bevorzugten Ausführungsform enthalten die erfindungsgemäßen Zweikomponentenmittel als Pflegestoff mindestens ein Vitamin, ein Provitamin, eine Vitaminvorstufe und/oder eines derer Derivate.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf die gesamte Anwendungszubereitung, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf die gesamte Anwendungszubereitung, enthalten. Mengen von 0,1 - 5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäßen Mitteln bevorzugt in Mengen von 0,01 - 5 Gew.-%, bezogen auf die gesamte Anwendungszubereitung, enthalten. Mengen von 0,05 -1 Gew.-% sind besonders bevorzugt.

Vitamin C (Ascorbinsäure). Vitamin C wird in den erfindungsgemäß verwendeten Mitteln bevorzugt in Mengen von 0,1 bis 3 Gew.-%, bezogen auf die gesamte Anwendungszubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf die gesamte Anwendungszubereitung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung enthalten.

Bevorzugt enthalten die erfindungsgemäßen Zweikomponentenmittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

Im Rahmen einer fünften bevorzugten Ausführungsform enthalten die erfindungsgemäßen Zweikomponentenmittel mindestens einen Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflegeund Waschmittel e.V. (lKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Ei bisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingeetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Im Rahmen einer sechsten Ausführungsform enthalten die erfindungsgemäßen Zweikomponentenmittel als Pflegestoff mindestens eine Carbonsäure.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäß einsetzbaren Carbonsäuren sind beispielsweise zu zählen C₁-C₈-Alkyl-, C₂-C₈-Alkenyl-, Aryl-, Aralkyl- und Aralkenyl-, Hydroxymethyl-, C₂-C₈-Hydroxyalkyl-,C₂-C₈-Hydroxyalkenyl-, Aminomethyl-, C₂-C₈-Aminoalkyl-, Cyano-, Formyl-, Oxo-, Thioxo-, Hydroxy-, Mercapto-, Amino-, Carboxy- oder Iminogruppen. Bevorzugte Substituenten sind C₁-C₈-Alkyl-, Hydroxymethyl-, Hydroxy-, Amino- und Carboxygruppen. Besonders bevorzugt sind Substituenten in α- Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäß einsetzbare Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxyphthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 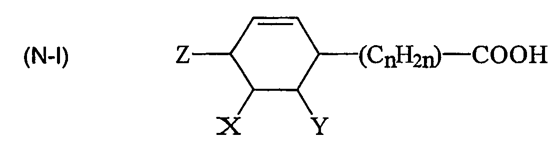 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt. So ist beispielweise der US-Patentschrift 3,753,968 ein Herstellungsverfahren zu entnehmen.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbindung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze, sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf die gesamte Anwendungszubereitung, besonders bevorzugt 0,1 bis 5 Gew.%, und insbesondere bevorzugt 0,1 bis 3 Gew.%.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, dass neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der ß-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Im Rahmen einer siebten bevorzugten Ausführungsform enthalten die erfindungsgemäßen Zweikomponentenmittel als Pflegestoff mindestens ein Proteinhydrolysat und/oder eines seiner Derivate.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{©} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in den erfindungsgemäßen Zweikomponentenmitteln beispielsweise in Konzentrationen von 0,01 Gew.% bis zu 20 Gew.%, vorzugsweise von 0,05 Gew.% bis zu 15 Gew.% und ganz besonders bevorzugt in Mengen von 0,05 Gew.% bis zu 5 Gew.%, jeweils bezogen auf die gesamte Anwendungszubereitung enthalten.

Im Rahmen einer achten bevorzugten Ausführungsform enthalten die erfindungsgemäßen Zubereitungen als Pflegestoff Ectoin oder Ectoinderivate, Allantoin, Taurin und/oder Bisabolol.

Erfindungsgemäß werden unter dem Begriff "Ectoin und Ectoinderivate" Verbindungen der Formel (IV) 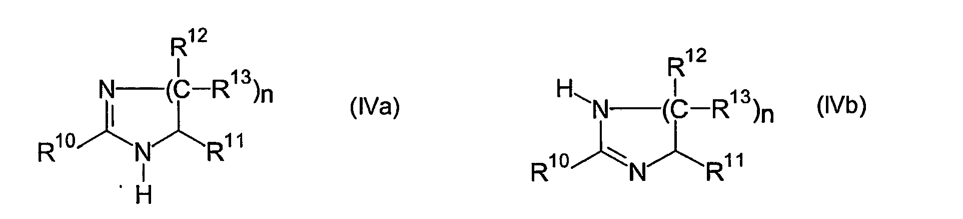 und/oder deren physiologisch verträglichen Salzes und/oder einer isomeren oder stereoisomeren Form verstanden, wobei
R¹⁰ steht für ein Wasserstoffatom, einen verzweigten oder unverzweigten C₁ - C₄-Alkylrest oder einen C₂ - C₄-Hydroxyalkylrest,
R¹¹ steht für ein Wasserstoffatom, eine Gruppierung -COOR¹⁴ oder eine Gruppierung - CO(NH)R¹⁴, wobei R¹⁴ für ein Wasserstoffatom, einen C₁ - C₄-Alkylrest, einen Aminosäurerest, einen Dipeptid- oder einen Tripeptidrest stehen kann,
R¹² und R¹³ stehen unabhängig voneinander für ein Wasserstoffatom, einen C₁ - C₄-Alkylrest oder eine Hydroxygruppe, mit der Maßgabe, dass nicht beide Reste gleichzeitig für eine Hydroxygruppe stehen dürfen, und
n steht für eine ganze Zahl von 1 bis 3.

Geeignete physiologisch verträgliche Salze der allgemeinen Verbindungen gemäß der Formel (IVa) oder (IVb) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (IVa) oder (IVb) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) C₁ - C₄- Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (IVa) oder (IVb), sowie die Salze, die sich durch Umsetzung von Verbindungen gemäß der Formel (IVa) oder (IVb) mit Salzsäure, Essigsäure, Citronensäure und Benzoesäure ergeben.

Unter isomeren oder stereoisomeren Formen der Verbindungen gemäß Formel (IVa) oder (IVb) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden: Asparagin, Arginin, Asparaginsäure, Glutamin, Glutaminsäure, β-Alanin, γ-Aminobutyrat, N_{ε}-Acetyllysin, N_{δ}-Acetylornitin, N_{γ} Acetyldiaminobutyrat, N_{α}-Acetyldiaminobutyrat, Histidin, Isoleucin, Leucin, Methionin, Phenylalanin, Serin, Threonin und Tyrosin. L-Aminosäuren sind bevorzugt. Aminosäurereste leiten sich von den entsprechenden Aminosäuren ab. Die folgenden Aminosäurereste sind bevorzugt: Gly, Ala, Ser, Thr, Val, β-Ala, γ-Aminobutyrat, Asp, Glu, Asn, Aln, N_{ε}-Acetyllysin, N_{δ}-Acetylornithin, N_{γ}-Acetyldiaminobutyrat, N_{α}-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für C₁ - C₄-Alkylgruppen in den erfindungsgemäßen Verbindungen sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte C₂ - C₄-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Die erfindungsgemäßen Zweikomponentenmittel enthalten diese Pflegestoffe bevorzugt in Mengen von 0,001 bis 2, insbesondere von 0,01 bis 0,5 Gew.-%, jeweils bezogen auf die gesamte Anwendungszubereitung.

Im Rahmen einer neunten bevorzugten Ausführungsform enthält die Zubereitung (B) als Pflegestoff mindestens ein Mono- bzw. Oligosaccharid.

Es können sowohl Monosaccharide als auch Oligosaccharide, wie beispielweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäßen Haarbehandlungsmitteln bevorzugt in einer Menge von 0,1 bis 8 Gew.-%, insbesondere bevorzugt 1 bis 5 Gew.-%, bezogen auf die gesamte Anwendungszubereitung, enthalten.

Im Rahmen einer zehnten Ausführungsform enthält die Zubereitung (B) als Pflegestoff mindestens ein Silikonöl und/oder ein Silikongum.

Erfindungsgemäß geeignete Silikone oder Silikongums sind insbesondere Dialkyl- und Alkylarylsiloxane, wie beispielsweise Dimethylpolysiloxan und Methylphenyl-polysiloxan, sowie deren alkoxylierte, quaternierte oder auch anionische Derivate.

Beispiele für solche Silikone sind:
- Oligomere Polydimethylcyclosiloxane (INCI-Bezeichnung: Cyclomethicone), insbesondere die tetramere und die pentamere Verbindung, die als Handelsprodukte DC 344 bzw. DC 345 von Dow Corning vertrieben werden,
- Hexamethyl-Disiloxan (INCI-Bezeichnung: Hexamethyldisiloxane), z. B. das unter der Bezeichnung Abil^{®} K 520 vertriebenen Produkt,
- Polymere Polydimethylsiloxane (INCI-Bezeichnung: Dimethicone), z. B. die unter der Bezeichnung DC 200 von Dow Corning vertriebenen Produkte,
- Polyphenylmethylsiloxane (INCI-Bezeichnung: Phenyl Trimethicone), z. B. das Handelsprodukt DC 556 Fluid von Dow Corning,
- Silicon-Glykol-Copolymere (INCI-Bezeichnung: Dimethicone Copolyol), z. B. die Handelsprodukte DC 190 und DC 193 von Dow Corning,
- Ester sowie Partialester der Silicon-Glykol-Copolymere, wie sie beispielsweise von der Firma Fanning unter der Handelsbezeichnung Fancorsil^{®} LIM (INCI-Bezeichnung: Dimethicone Copolyol Meadowfoamate) vertrieben werden,
- Dimethylsiloxane mit Hydroxy-Endgruppen (INCI-Bezeichnung: Dimethiconol), z. B. die Handelsprodukte DC 1401 und Q2-1403 von Dow Corning,
- aminofunktionelle Polydimethylsiloxane und hydroxylaminomodifizierte Silicone (INCI-Bezeichnung: u. a. Amodimethicone und Quaternium-80), wie die Handelsprodukte XF42-B1989 (Hersteller GE Toshiba Silicones) Q2-7224 (Hersteller: Dow Corning; ein stabilisiertes Trimethylsilylamodimethicon), Dow Corning^{®} 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt),
- anionische Silikonöle, wie beispielsweise das Produkt Dow Corning^{®}1784
- aminomodifizierte Organosilicone, wie beispielsweise das Produkt Abil Soft A843 (Hersteller Osi Specialities).

Gemäß einer bevorzugten Ausführungsform enthalten die erfindungsgemäßen Zubereitungen eine Kombination aus einem flüchtigen und einem nichtflüchtigen Silikon. Flüchtig im Sinne der Erfindung sind solche Silikone, die ein Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning^{®}1401, Dow Corning^{®}1403 und Dow Corning^{®}1501, jeweils Mischungen aus einem Cyclomethicone und einem Dimethiconol) erhältlich.

Gemäß einer besonders bevorzugten Ausführungsform enthält die Zubereitung (B) als Pflegestoff ein Dialkylpolysiloxan oder eines seiner Derivate. Bevorzugt sind die Alkylgruppen Methyl, Ethyl, i-Propyl und n-Propyl. Dimethylpolysiloxan oder eines seiner Derivate wird besonders bevorzugt eingesetzt. Bevorzugt sind die Derivate des Dimethylpolysiloxans, die aminofunktionell sind. Ein ganz besonders bevorzugtes Derivat ist unter der INCI-Bezeichnung Amodimethicone im Handel erhältlich.

Die erfindungsgemäßen Zubereitungen enthalten die Silikone bevorzugt in Mengen von 0,01 - 10 Gew.-%, insbesondere 0,1 - 5 Gew.-%, bezogen auf die gesamte Anwendungszubereitung.

Im Rahmen einer elften Ausführungsform enthält die Zubereitung (B) mindestens ein Lipid als Pflegestoff.

Erfindungsgemäß geeignete Lipide sind Phospholipide, beispielsweise Sojalecithin, EiLecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Die erfindungsgemäßen Zubereitungen enthalten die Lipide bevorzugt in Mengen von 0,01 - 10 Gew.-%, insbesondere 0,1 - 5 Gew.-%, bezogen auf die gesamte Anwendungszubereitung.

Im Rahmen einer zwölften Ausführungsform enthält die Zubereitung (B) mindestens einen Ölkörper als Pflegestoff.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyln-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀- Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 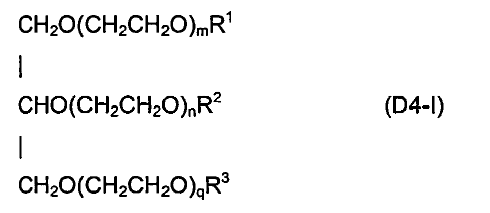 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono-und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Zweikomponentenmittein beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Im Rahmen einer dreizehnten Ausführungsform enthält die Zubereitung (B) ein Enzym als Pflegestoff. Erfindungsgemäß besonders bevorzugte Enzyme sind ausgewählt aus einer Gruppe, die gebildet wird aus Proteasen, Lipasen, Transglutaminase, Oxidasen und Peroxidasen.

Im Rahmen einer vierzehnten Ausführungsform enthalten die erfindungsgemäßen Zweikomponentenmittel mindestens einen Perlenextrakt.

Perlen von Muscheln bestehen im wesentlichen aus anorganischen und organischen Calciumsalzen, Spurenelementen und Proteinen. Perlen lassen sich auf einfache Weise aus kultivierten Muscheln gewinnen. Die Kultivierung der Muscheln kann sowohl in Süßwasser als auch in Meereswasser erfolgen. Dies kann sich auf die Inhaltsstoffe der Perlen auswirken. Erfindungsgemäß bevorzugt ist ein Perlenextrakt, welcher von in Meeres- bzw. Salzwasser kultivierten Muscheln stammt. Die Perlen bestehen zu einem großen Teil aus Aragonit (Calciumcarbonat), Conchiolin und einem Albuminoid. Letztere Bestandteile sind Proteine. Weiterhin sind in Perlen noch Magnesium- und Natriumsalze, anorganische Siliciumverbindungen sowie Phosphate enthalten.

Zur Herstellung des Perlenextraktes werden die Perlen pulverisiert. Danach werden die pulverisierten Perlen mit den üblichen Methoden extrahiert. Als Extraktionsmittel zur Herstellung der Perlenextrakte können Wasser, Alkohole sowie deren Mischungen verwendet werden. Unter Wasser sind dabei sowohl demineralisiertes Wasser, als auch Meereswasser zu verstehen. Unter den Alkoholen sind dabei niedere Alkohole wie Ethanol und Isopropanol, insbesondere aber mehrwertige Alkohole wie Glycerin, Diglycerin, Triglycerin, Polyglycerin, Ethylenglykol, Propylenglykol und Butylenglykol, sowohl als alleiniges Extraktionsmittel als auch in Mischung mit demineralisiertem Wasser oder Meereswasser, bevorzugt. Perlenextrakte auf Basis von Wasser/Glyceringemischen haben sich als besonders geeignet erwiesen. Je nach Extraktionsbedingungen können die Perlenproteine (Conchiloin und Albuminoid) weitestgehend in nativem Zustand oder bereits teilweise oder weitestgehend als Proteinhydrolysate vorliegen. Bevorzugt ist ein Perlenextrakt, in welchem Conchiolin und Albuminoid bereits teilweise hydrolysiert vorliegen. Die wesentlichen Aminosäuren dieser Proteine sind Glutaminsäure, Serin, Alanin, Glycin Asparaginsäure und Phenylalanin. In einer weiteren besonders bevorzugten Ausgestaltung kann es vorteilhaft sein, wenn der Perlenextrakt zusätzlich mit mindestens einer oder mehreren dieser Aminosäuren angereichert wird. In der bevorzugtesten Ausführungsform ist der Perlenextrakt angereichert mit Glutaminsäure, Serin und Leucin. Weiterhin findet sich je nach Extraktionsbedingungen, insbesondere in Abhängigkeit von der Wahl des Extraktionsmittels ein mehr oder weniger großer Anteil an Mineralien und Spurenelementen im Extrakt wieder. Ein bevorzugter Extrakt enthält organische und/oder anorganische Calciumsalze sowie Magnesium- und Natriumsalze, anorganische Siliciumverbindungen und/oder Phosphate. Ein ganz besonders bevorzugter Perlenextrakt enthält mindestens 75 %, bevorzugt 85 %, besonders bevorzugt 90 % und ganz besonders bevorzugt 95 % aller Inhaltsstoffe der natürlich vorkommenden Perlen. Beispiele für erfindungsgemäß einsetzbare Perlenextrakte sind die Handelsprodukte Pearl Protein Extract BG^{®} oder Crodarom^{®} Pearl.

Die zuvor beschriebenen Perlenextrakte sind vorzugsweise in einer Menge von mindestens 0,01 bis zu 20 Gew.% enthalten. Bevorzugt werden Mengen des Extraktes von 0,01 bis zu 10 Gew.%, ganz besonders bevorzugt Mengen von 0,01 bis 5 Gew.% bezogen auf das gesamte Zweikomponentenmittel verwendet.

Obwohl jeder der in den verschiedenen Ausführungsformen genannten Pflegestoffe für sich alleine bereits ein zufriedenstellendes Resultat ergibt, sind im Rahmen der vorliegenden Erfindung auch alle Ausführungsformen umfasst, in denen die Zubereitung (B) mehrere Pflegestoffe auch aus verschiedenen Gruppen enthält.

Das erfindungsgemäße Zweikomponentenmittel ist in verschiedenen Kammern einer Zweikammertube konfektioniert, wobei eine erste Kammer die Zubereitung (A) und eine zweite Kammer die Zubereitung (B) aufnimmt. Es ist aber auch möglich, eine Mehrkammertube einzusetzen, die mehr als zwei Kammern, beispielsweise drei oder vier Kammern, aufweist. In diesem Fall kann Zubereitung (A) und/oder Zubereitung (B) auf mehrere Kammern der Mehrkammertube verteilt werden, wobei natürlich darauf zu achten ist, dass sich in einer bestimmten Kammer ausschließlich Zubereitung (A) oder Zubereitung (B) befindet.

Zweikammertuben sind bereits im Stand der Technik bekannt. In einer besonders einfachen Ausführungsform haben die Tuben zwei getrennte Kammern, die als ineinandergesteckte Schläuche ausgebildet sind. Diese definieren die innere und die äußere Kammer und enden in dem gemeinsamen Kopf- oder Austrittsbereich. Der Kopfbereich ist derart gestaltet, dass die beiden Zubereitungen gemeinsam aus der Tube austreten, sobald Druck auf diese ausgeübt wird. Durch die Gestaltung des Kopfbereiches wird bestimmt, in welchem Streifenmuster die Zubereitungen aus der Tu be austreten. Die bekannten handelsüblichen Tuben weisen ein gleiches Verhältnis der Volumina der Innentube zur Außentube und somit ein Mischungsverhältnis von 50 : 50 auf. Für Produkte, deren zwei Phasen getrennt aufbewahrt werden müssen und deren Mischungsverhältnis von dem herkömmlichen Wert von 50 : 50 abweicht, sind die bekannten Tuben nicht tauglich.

Die erfindungsgemäßen Zweikomponentenmittel werden in einer Zwekammertube konfektioniert, die eine innere und einer äußere Kammer aufweist, die beide in einem gemeinsamen Kopfbereich (Austrittsbereich) enden. Der Kopfbereich ist derart gestaltet, dass die beiden Zubereitungen gemeinsam aus der Tube austreten, sobald Druck auf diese ausgeübt wird. Durch die Gestaltung dieses Kopfbereiches wird bestimmt, in welchem Muster die Zubereitungen aus der Tube austreten.

Die Wahl der Volumina der einzelnen Kammern richtet sich nach dem gewünschten Verhältnis der Volumina von Zubereitung (A) und Zubereitung (B) im Zweikomponentenmittel. In der Regel sind die Volumina in einem Verhältnis vorgesehen, das sich von der bislang bekannten gleichmäßigen Aufteilung unterscheidet. Dabei soll unter nicht gleichmäßiger Aufteilung jegliche ungleiche Verteilung der Volumina verstanden werden, die einen signifikanten Unterschied aufweist.

Neben dem Merkmal der unterschiedlichen Kammervolumina zeichnet sich die bevorzugt eingesetzte Zweikammertube durch eine besondere Gestaltung des Austrittsbereiches aus. Auch dort spiegelt sich das Verhältnis der Kammervolumina in den Querschnitten der für die Teilströme definierten Wege wieder. Dabei sei darauf hingewiesen, dass der Teilstrom einer Zubereitung mehrere parallele Zweigströme aufweisen kann. So können Trennmittel den Querschnitt des Durchgangskanals zumindest nahezu entsprechend dem Verhältnis in zwei oder mehr Teilströme aufteilen. Dabei ist anzumerken, dass es für die Funktion der Zweikammertuben vorteilhaft ist, wenn die in den jeweiligen Tubenkammern vorhandenen verschiedenen Komponenten jeweils etwa die gleiche Viskosität aufweisen. Zum Erhalt der rezepturbedingten Mischungsverhältnisse und zum gleichmäßigen Produktaustritt sind Geometrien der Tubenöffnungen vorteilhaft, die ein Mischungsverhältnis ungleich 50:50, nämlich von 80 : 20 bis 60 : 40, vorzugsweise 75 : 25, aufweisen.

Obwohl die Erfindung prinzipiell hinsichtlich des Musters, mit dem die Zubereitungen aus der Tube austreten, in keiner Weise beschränkt sein soll, kann es erfindungsgemäß bevorzugt sein, wenn die erste Zubereitung als Hauptstrang austritt und die zweite Zubereitung mehrere an diesem Hauptstrang entlanglaufende Streifen bildet. Auch hinsichtlich der Zahl dieser Streifen soll die Erfindung nicht eingeschränkt sein. Eine Zahl von 2 bis 4 Streifen kann erfindungsgemäß aus applikationstechnischen Gründen aber besonders bevorzugt sein. Dabei kann in einer ersten Ausgestaltungsform die Zubereitung (A) die Streifen bilden, während die Zubereitung (B) den Hauptstrang bildet und in einer zweiten Ausgestaltungsform die Zubereitung (B) die Streifen bilden, während die Zubereitung (A) den Hauptstrang bildet.

In einer weiteren Ausgestaltungsform, kann es aber auch bevorzugt sein, wenn die beiden Zubereitungen anteilig gemeinsam nebeneinander den Hauptstrang bilden. In einer weiteren Ausgestaltungsform kann der Austrittsstrang aus einem inneren Bereich, gebildet aus einer ersten Zubereitung, und einem äußeren Bereich, gebildet aus der zweiten Zubereitung, bestehen, wobei die Zubereitungen entsprechend ihrer Anordnung in der Tube auch den Austrittsstrang bilden.

Das Mengenverhältnis der Zubereitung (A) zur Menge der Zubereitung (B) liegt erfindungsgemäß bevorzugt in einem Bereich von 1:2 bis 5:1. Ein Mengenverhältnis von 2:1 bis 3:1 hat sich als ganz besonders bevorzugt erwiesen.

In der erfindungsgemäßen Ausführungsform besteht die Zweikammertube aus einer inneren Tube, die von einer äußeren Tube vollständig umgeben ist. Diese Ausführungsform zeichnet sich durch eine optimal gleichbleibende Dosierung der beiden Zubereitungen aus. Obwohl prinzipiell jede Verteilung der Zubereitungen auf die Kammern der Tube erfindungsgemäß umfasst sein soll, kann es besonders bevorzugt sein, wenn die Zubereitung (A) sich in der Außentube befindet, und die Zube reitung (B) sich in der Innentube befindet.

Die Zweikammertube ist vorzugsweise aus einem Material gefertigt, das zur Verpackung von Tönungs- und Färbemitteln dieser Art geeignet ist. Als erfindungsgemäß besonders geeignet hat sich sowohl für die Außenwände als auch für die Innenwände laminiertes Aluminium erwiesen. Es sind aber auch Tuben aus Kunststofflaminat (PE, PET, PP) oder Kunststoffcoextrudaten (PE, PET, PP) denkbar. Darüber hinaus kann in einer Ausführungsform das Material der Innentube unabhängig von dem Material der Außentube gewählt werden. Als erfindungsgemäß ganz besonders bevorzugt hat sich eine Tube erwiesen, bei der sowohl die Innentube als auch die Außentube aus Aluminiumlaminat gefertigt ist. Unter Aluminiumlaminat wird erfindungsgemäß eine mit Kunststoff beschichtete Aluminiumschicht verstanden.

Im Hinblick darauf, dass bei einem Tönungsmittel der Anteil der Farbzubereitung etwa 75% und der Anteil der Pflegezubereitung etwa 25% ausmachen kann und daher die Farbzubereitung vorteilhafter Weise in die Außentube mit dem größeren Volumen eingebracht wird, ist es besonders vorteilhaft, wenn der Schulterbereich der Außentube mit Ronden verstärkt wird, die besonders gute Barriereeigenschaften aufweisen. Dabei ist es vorteilhaft, in das Material der Ronden Aluminium einzuarbeiten.

Um ein Austreten der Mischung während der Lagerung zu verhindern und dem Verbraucher die Unversehrtheit der Tube zu versichern, ist es vorteilhaft, die Ausgabeöffnung mit einem Originalitätsverschluss aus Aluminium oder Kunststoff zu versiegeln, der vom Verbraucher entfernt wird.

Neben den wesentlichen Komponenten können die Zubereitungen (A) und (B) weiterhin alle für solche Zubereitungen bekannten Wirk-, Zusatz- und Hilfsstoffe enthalten. In vielen

Fällen enthalten die Zubereitungen mindestens ein Tensid, wobei prinzipiell sowohl anionische als auch zwitterionische, ampholytische und nichtionische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹ O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-₁₈-Acylsarcosin.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Zubereitungen weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genußsäuren und Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Konservierungsmittel,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Zubereitungen enthalten die erfindungswesentlichen Komponenten bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos oder andere Zubereitungen, die für die Anwendung auf dem Haar geeignet sind.

Unter wässrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Weiterhin können die erfindungsgemäßen Zweikomponentenmittel ein Reduktionsmittel enthalten. Beispiele für erfindungsgemäß bevorzugte Reduktionsmittel sind Natriumsulfit, Ascorbinsäure, Thioglykolsäure und deren Derivate, Natriumthionit, Alkalimetallcitratsalze und N-Acetyl-L-Cystein. Ganz besonders bevorzugte Reduktionsmittel sind Alkalimetallcitratsalze, insbesondere Natriumcitrat, und N-Acetyl-L-Cystein. N-Acetyl-L-Cystein ist ein ganz besonders bevorzugtes Reduktionsmittel.

Weiterhin können die erfindungsgemäßen Mittel Alkalisierungsmittel, üblicherweise Alkalioder Erdalkalihydroxide, Ammoniak oder organische Amine, enthalten. Bevorzugte Alkalisierungsmittel sind Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Weiterhin können die erfindungsgemäßen Zweikomponentenmittel in der Zubereitung (A) und/oder der Zubereitung (B) zur Anfärbung Perlglanzpigmente enthalten. Erfindungsgemäß bevorzugte Perlglanzpigmente sind natürliche Perlglanzpigmente wie z.B. Fischsilber (Guanin/Hypoxanthin-Mischkristalle aus Fischschuppen) oder Perlmutt (aus vermahlenen Muschelschalen), monokristalline Perlglanzpigmente wie z.B. Bismutoxychlorid, sowie Perlglanzpigmente auf Basis von Glimmer oder Glimmer/Metalloxid. Letztgenannte Perlglanzpigmente werden mit einem Metalloxidcoating versehen. Durch den Einsatz der Perlglanzpigmente werden Glanz und gegebenenfalls zusätzlich Farbeffekte in den erfindungsgemäßen Zweikomponentenmitteln erzielt. Die Farbgebung durch die in den Zweikomponentenmitteln verwendeten Perlglanzpigmente beeinflusst das Farbergebnis der Färbung der Keratinfasern jedoch nicht.

Perlglanzpigmente auf Glimmer-Basis und auf Glimmer/Metalloxid-Basis sind erfindungsgemäß ebenfalls bevorzugt. Glimmer gehören zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet. Geeignete Metalloxide sind u.a. TiO₂, Cr₂O₃ und Fe₂O₃. Durch entsprechende Beschichtung werden Interferenzpigmente sowie Farbglanzpigmente als erfindungsgemäße Perlglanzpigmente erhalten. Diese Perlglanzpigmentarten weisen neben einem glitzernden optischen Effekt zusätzlich Farbeffekte auf. Des weiteren können die erfindungsgemäß verwendbaren Perlglanzpigmente weiterhin ein Farbpigment enthalten, welches sich nicht von einem Metalloxid ableitet.

Die Korngröße der bevorzugt verwendeten Perlglanzpigmente liegt bevorzugt zwischen 1.0 und 100 µm, besonders bevorzugt zwischen 5.0 und 60.0 µm.

Besonders bevorzugte Perlglanzpigmente sind Pigmente, die von der Firma Merck unter den Handelsnamen Colorona^{®} vermarktet werden, wobei die Pigmente Colorona^{®} redbrown (47-57 Gew.% Muscovit Mica (KH₂(AlSiO₄)₃), 43-50 Gew.% Fe₂O₃ (INCI: Iron Oxides Cl 77491), <3 Gew.% TiO₂ (INCI: Titanium Dioxide Cl 77891), Colorona^{®} Blackstar Blue (39-47 Gew.% Muscovit Mica (KH₂(AlSiO₄)₃), 53-61 Gew.% Fe₃O₄ (INCI: Iron Oxides Cl 77499)), Colorona^{®} Siena Fine (35-45 Gew.% Muscovit Mica (KH₂(AlSiO₄)₃), 55-65 Gew.% Fe₂O₃ (INCI: Iron Oxides Cl 77491 )), Colorona^{®} Aborigine Amber (50-62 Gew.% Muscovit Mica (KH₂(AlSiO₄)₃), 36-44 Gew.% Fe₃O₄ (INCI: Iron Oxides Cl 77499), 2-6 Gew.% TiO₂ (INCI: Titanium Dioxide Cl 77891)), Colorona^{®} Patagonian Purple (42-54 Gew.% Muscovit Mica (KH₂(AlSiO₄)₃), 26-32 Gew.% Fe₂O₃ (INCI: Iron Oxides Cl 77491), 18-22 Gew.% TiO₂ (INCI: Titanium Dioxide Cl 77891), 2-4 Gew.% Preussisch Blau (INCI: Ferric Ferrocyanide Cl 77510)), Colorona^{®} Chameleon (40-50 Gew.% Muscovit Mica (KH₂(AlSiO₄)₃), 50-60 Gew.% Fe₂O₃ (INCI: Iron Oxides Cl 77491)) und Silk^{®} Mica (>98 Gew.% Muscovit Mica (KH₂(AlSiO₄)₃)) ganz besonders bevorzugt sind.

Bezüglich der in den erfindungsgemäßen Zweikomponentenmitteln einsetzbaren Perlglanzpigmente wird weiterhin ausdrücklich auf die Monographien Inorganic Pigments, Chemical technology review Nr. 166, 1980, Seiten 161-173 (ISBN 0-8155-0811-5) und Industrial inorganic Pigments, 2. Auflage, Weinheim, VCH, 1998, Seiten 211-231, Bezug genommen.

Falls die Zubereitung (A) eine Vorstufe eines naturanalogen Farbstoffes enthält, kann die oxidative Farbentwicklung grundsätzlich mit Luftsauerstoff erfolgen. In einer speziellen Ausführungsform dieses Gegenstandes kann die austretende Anwendungszubereitung aber auch unmittelbar vor der Anwendung noch mit einer Zubereitung, enthaltend ein Oxidationsmittel, vermischt werden. Als Oxidationsmittel kommen Persulfate, Chlorite und insbesondere Wasserstoffperoxid oder dessen Anlagerungsprodukte an Harnstoff, Melamin sowie Natriumborat in Frage. Bevorzugt wird jedoch auf den Zusatz eines chemischen Oxidationsmittels verzichtet.

Das eigentliche Tönungs- und/oder Färbemittel wird durch Durchmischung der beiden aus der Tube ausgetretenen Zubereitungen (A) und (B) erhalten. Diese Durchmischung der getrennt aus der Tube austretenden Zubereitungen (A) und (B) kann sowohl vor der Anwendung auf den Fasern in einem separaten Schritt als auch als Nebeneffekt bei der Einarbeitung der Austrittsstranges in die Fasern erfolgen. Das dabei entstehende gebrauchsfertige Präparat sollte bevorzugt einen pH-Wert im Bereich von 6 bis 12, insbesondere von 8 bis 10, aufweisen. Sofern nichts anderes vermerkt ist, ist unter den Angaben zum pH-Wert im Rahmen der vorliegenden Offenbarung der pH-Wert bei 25°C zu verstehen. Besonders bevorzugt ist die Anwendung der Haarfärbemittel in einem schwach alkalischen Milieu. Die Anwendungstemperaturen können in einem Bereich zwischen 15 und 40°C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die erfindungsgemäßen Zubereitungen (A) und (B) weisen vorzugsweise Viskositäten im Bereich von 2 000 bis 200 000 mPas, insbesondere von 5 000 bis 50 000 mPas (Brookfield-Viskosimeter, Spindel Nr. 4, 20 rpm, 20°C) auf. Auf diese Weise ist gewährleistet, dass das Zweikomponentenmittel eine gute Mischbarkeit aufweist und dennoch das Austrittsmuster eine hinreichende Stabilität aufweist.

Ein weiterer Gegenstand der vorliegenden Erfindung ist ein Verfahren zur Tönung von keratinischen Fasern, insbesondere menschlichen Haaren, wobei ein erfindungsgemäßes Zweikomponentenmittel aus der Tube herausgedrückt wird, die Anwendungszubereitung auf die Fasern aufgebracht wird und nach einer Einwirkungszeit wieder abgespült wird.

Die folgenden Beispiele sollen den Gegenstand der vorliegenden Anmeldung erläutern ohne ihn zu beschränken.

### Ausführungsbeispiele:

Es wurden die folgenden Rezepturen hergestellt. Die Mengenangaben verstehen sich - soweit nichts anderes vermerkt ist - in Gewichtsprozent.

Rezepturen der Zubereitungen (A)

| Rohstoff | Tönungscreme 1 (Kupferblond) | Tönungscreme 2 (Violett) |
|---|---|---|
| Natriumlaurethsulfat, 27%ig in H₂O | 8,00 | 8,00 |
| Wässrige Ammoniumcarbopollösung, 1%ig | 35,00 | 35,00 |
| Lowenol^{®} C-279 | 0,50 | -- |
| Cetylalkohol | 2,00 | 2,00 |
| Polawax^{®} GP 200 | 1,50 | 1,50 |
| Lanette^{®} 14 | 1,20 | 1,20 |
| Paraffinoel DAB9 | 1,00 | 1,00 |
| Emulgade^{®} 1000NI | 3,50 | 3,50 |
| Ethyldiglykol | 1,00 | 1,00 |
| Methoxybutanol | 5,00 | -- |
| Benzylalkohol | 0,50 | 1,00 |
| Tetrasodium EDTA | 0,15 | 0,15 |
| HC Red No.3 | 0,70 | 0,60 |
| Violet 1,4 D | 0,01 | -- |
| HC Yellow No.2 | 0,30 | -- |
| 6-Chloro-4-nitro-2-aminophenol | 0,15 | -- |
| HC Blue No. 2 | -- | 0,6 |
| Basic Blue 99 | -- | 0,1 |
| 2-Amino-2-methyl-propanol | ad pH 10 | ad pH 10 |
| Parfüm | 0,20 | 0,20 |
| Wasser | ad 100 | ad 100 |

| Rohstoff | Tönungscreme 3 (Caramel) | Tönungscreme 4 (Rotbraun) |
|---|---|---|
| Cetylstearylalkohol | 8,00 | 8,00 |
| Glycerinmonostearat | 1,50 | 1,50 |
| Isopropylmyristat | 3,50 | 3,50 |
| Laureth-23 | 0,40 | 0,40 |
| Ceteareth-30 | 1,00 | 1,00 |
| Lanette^{®} 14 | 1,20 | 1,20 |
| Cutina^{®} HR | 1,00 | 1,00 |
| Ethyldiglykol | 1,00 | 1,00 |
| Methoxybutanol | 5,00 | -- |
| Benzylalkohol | 0,50 | 1,00 |
| Tetrasodium EDTA | 0,15 | 0,15 |
| HC Red No.3 | 0,01 | 0,6 |
| HC Yellow No. 12 | 0,15 | -- |
| HC Yellow No.2 | 0,10 | 0,20 |
| 6-Chloro-4-nitro-2-aminophenol | 0,06 | 0,08 |
| HC Blue No. 2 | 0,50 | 0,40 |
| Basic Blue 99 | -- | 0,01 |
| N,N-Bis(2-hydroxyethylmethyl)amino-5-nitrobenzol | -- | 0,15 |
| 2-Amino-2-methyl-propanol | ad pH 10 | ad pH 10 |
| Parfüm | 0,20 | 0,20 |
| Wasser | ad 100 | ad 100 |

| Rohstoff | Tönungscreme 5 (Rot) | Tönungscreme 6 (Braun) |
|---|---|---|
| Cetylstearylalkohol | 8,00 | 8,00 |
| Covasterol | 0,50 | 0,50 |
| Isopropylmyristat | 3,40 | 3,40 |
| Laureth-23 | 0,30 | 0,30 |
| Cutina^{®} HR | 0,80 | 0,80 |
| Myristylalkohol | 1,00 | 1,00 |
| Ceteareth-30 | 1,00 | 1,00 |
| Propylenglykol | 4,00 | 3,00 |
| Benzylalkohol | 0,50 | 0,50 |
| Tetrasodium EDTA | 0,30 | 0,30 |
| HC Red No.3 | 1,30 | -- |
| Violet 1,4 D | 0,002 -- | -- |
| N,N-Bis(2-hydroxyethyl)-2-nitro-p-phenylendiamin | -- | 1,00 |
| 4-Amino-3-nitrophenol | -- | 0,02 |
| HC Blue No.2 | -- | 1,50 |
| Basic Blue 99 | -- | 0,50 |
| HC Yellow No.2 | -- | 0,10 |
| 2-Amino-2-methyl-propanol | ad pH 9,5 | ad pH 9,5 |
| Parfüm | 0,30 | 0,30 |
| Wasser | ad 100 | ad 100 |

| Rohstoff | Tönungscreme 7 (Kupfer) | Tönungscreme 8 (Aschblond) |
|---|---|---|
| Cetylstearylalkohol | 8,00 | 8,00 |
| Covasterol | 0,50 | 0,50 |
| Isopropylmyristat | 3,40 | 3,40 |
| Laureth-23 | 0,30 | 0,30 |
| Cutina^{®} HR | 0,80 | 0,80 |
| Myristylalkohol | 1,00 | 1,00 |
| Ceteareth-30 | 1,00 | 1,00 |
| Cetyltrimethylammoniumchlorid, 25%ig | 9,00 | 9,00 |
| Propylenglykol | 4,00 | 2,00 |
| Benzylalkohol | 0,80 | 0,30 |
| Tetrasodium EDTA | 0,30 | -- |
| N,N Bis(2-hydroxyethyl)-2-nitro-p-phenylendiamin | 0,02 | -- |
| HC Blue No.2 | -- | 0,05 |
| HC Yellow No.2 | 0,30 | -- |
| 2-Amino-6-chlor-4-nitrophenol | 0,20 | -- |
| 4-Hydroxypropylamino-3-nitrophenol | 0,50 | -- |
| 2-Amino-2-methyl-propanol | ad pH 9,0 | ad pH 9,5 |
| Parfüm | 0,20 | 0,40 |
| Wasser | ad 100 | ad 100 |

| Rohstoff | Tönungscreme 9 (Orange) | Tönungscreme 10 (Rehbraun) |
|---|---|---|
| Cetylstearylalkohol | 8,00 | 8,00 |
| Covasterol | 0,50 | 0,50 |
| Isopropylmyristat | 3,40 | 3,40 |
| Laureth-23 | 0,30 | 0,30 |
| Cutina^{®} HR | 0,80 | 0,80 |
| Myristylalkohol | 1,00 | 1,00 |
| Ceteareth-30 | 1,00 | 1,00 |
| Cetyltrimethylammoniumchlorid, 25%ig | 9,00 | 9,00 |
| Propylenglykol | -- | 3,00 |
| Benzylalkohol | 0,50 | 0,50 |
| Tetrasodium EDTA | 0,30 | 0,30 |
| HC Blue No.2 | -- | 0,80 |
| HC Yellow No.2 | -- | 0,20 |
| 2-Amino-6-chlor-4-nitrophenol | -- | 0,15 |
| HC Red No.15 | -- | 0,20 |
| HC Yellow No.13 | 0,02 | 0,05 |
| Basic Yellow 87 | 0,20 | -- |
| Basic Orange 31 | 0,05 | -- |
| 2-Amino-2-methyl-propanol | ad pH 9,0 | ad pH 9,5 |
| Parfüm | 0,30 | 0,30 |
| Wasser | ad 100 | ad 100 |

| Rohstoff | Tönungscreme 11 (Gelb) |
|---|---|
| Cetylstearylalkohol | 8,00 |
| Covasterol | 0,50 |
| Isopropylmyristat | 3,40 |
| Laureth-23 | 0,30 |
| Cutina^{®} HR | 0,80 |
| Myristylalkohol | 1,00 |
| Ceteareth-30 | 1,00 |
| Cetyltrimethylammoniumchlorid, 25%ig | 9,00 |
| Propylenglykol | 3,00 |
| Benzylalkohol | 0,80 |
| Tetrasodium EDTA | 0,30 |
| Basic Orange 31 | 0,01 |
| Basic Red 51 | 0,20 |
| 2-Amino-2-methyl-propanol | ad pH 9,5 |
| Parfüm | 0,30 |
| Wasser | ad 100 |

| Rezepturen der Zubereitungen (B) | | |
|---|---|---|
| Rohstoff | Pflegecreme A | Pflegecreme B |
| Natriumlaurethsulfat, 27%ig in H₂O | 8,00 | 8,00 |
| Wässrige Ammoniumcarbopollösung, 1 %ig | 35,00 | 35,00 |
| Cetylalkohol | 2,00 | 2,00 |
| Polawax^{®} GP 200 | 1,50 | 1,50 |
| Lanette^{®} 14 | 1,20 | 1,20 |
| Paraffinoel DAB9 | 1,00 | 1,00 |
| Emulgade^{®} 1000NI | 3,50 | 3,50 |
| Merquat^{®} 100 | 1,00 | 1,00 |
| Panthenol | -- | 0,50 |
| Crosilk^{®} Liquid | 0,50 | -- |
| 2-Amino-2-methyl-propanol | ad pH 9 | -- |
| Citronensäure | -- | ad pH 6,5 |
| Propylenglykol | -- | 4,00 |
| p-Hydroxybenzoesäuremethylester | -- | 0,15 |
| Parfüm | 0,20 | -- |
| Wasser | ad 100 | ad 100 |

| Rohstoff | Pflegecreme C | Pflegecreme D |
|---|---|---|
| Cetylstearylalkohol | 8,00 | 8,00 |
| Glycerinmonostearat | 1,50 | 1,50 |
| Isopropylmyristat | 3,50 | 3,50 |
| Ceteareth-30 | 1,00 | 1,00 |
| Lanette^{®} 14 | 1,20 | 1,20 |
| Cutina^{®} HR | 1,00 | -- |
| Merquat^{®} 100 | 1,00 | 1,00 |
| Panthenol | -- | 0,50 |
| Vitamin B6 | 0,50 | -- |
| 2-Amino-2-methylpropanol | ad pH 9 | -- |
| Citronensäure | -- | ad pH 6,5 |
| Propylenglykol | -- | 4,00 |
| p-Hydroxybenzoesäuremethylester | -- | 0,15 |
| MICA | -- | 0,10 |
| Parfüm | 0,20 | -- |
| Wasser | ad 100 | ad 100 |

| Rohstoff | Pflegecreme E | Pflegecreme F | Pflegecreme G |
|---|---|---|---|
| Cetylstearylalkohol | 6,50 | 8,00 | 7,00 |
| Covasterol | 0,30 | 0,40 | 0,40 |
| Isopropylmyristat | 2,90 | 3,00 | 3,00 |
| Laureth-23 | 0,20 | 0,25 | 0,20 |
| Cutina^{®} HR | 0,65 | 0,80 | 0,70 |
| Myristylalkohol | 0,80 | 0,90 | 0,90 |
| Ceteareth-30 | 0,80 | 0,90 | 0,90 |
| Cetyltrimethylammoniumchlorid, 25%ig | 9,00 | 9,00 | 9,00 |
| Propylenglykol | -- | -- | 1,00 |
| Tetrasodium EDTA | 0,30 | -- | 0,30 |
| Vitamin B6 | 1,00 | -- | -- |
| Merquat^{®} Plus 3330 | -- | 1,00 | -- |
| Serin | -- | -- | 1,00 |
| 2-Amino-2-methyl-propanol | ad pH 8,0 | ad pH 9,0 | ad pH 8,0 |
| Parfüm | 0,20 | 0,20 | -- |
| Wasser | ad 100 | ad 100 | ad 100 |

### Ausfärbungen:

Die Tönungscremes 1 und 2 wurden jeweils zusammen mit Pflegecreme A beziehungsweise B im Verhältnis 3:1 in einer Zweikammertube konfektioniert. Dabei enthielt die Innentube die Zubereitung (B) und die Außentube die Zubereitung (A). Die gesamte Tube bestand aus Aluminiumlaminat.

Die resultierenden Zweikomponentenmittel wurden jeweils aus den Tuben herausgedrückt und direkt auf Humanhaar (Fa. Kerling, Naturweiß) aufgebracht. Die Anwendungszubereitung wurde in das Haar einmassiert, dort für 30 min bei Raumtemperatur belassen und anschließend ausgespült. Nach Trocknung der Haare wurden intensive Kupfer- bzw. Violett-Nuancen erhalten.

Analog wurde mit den Tönungscremes 3 und 4 und den Pflegecremes C beziehungsweise D verfahren. Dabei wurden intensive Caramel- bzw. Rotbraun-Nuancen erhalten.

Ebenfalls analog wurde mit den Tönungscremes 5 bis 11 und den Pflegecremes E, F beziehungsweise G verfahren. Die erhaltenen Farbnuancen sind obigen Tabellen zu entnehmen.

### Verzeichnis der eingesetzten Handelsprodukte:

Die im Rahmen der Beispiele eingesetzten Handelsprodukte sind wie folgt definiert:
- Carbopol: Polyacrylsäure (INCl-Bezeichnung: Carbomer) (Noveon)
- Covasterol: (INCl-Bezeichnung: Glyceryl Isostearate, Isostearyl Alcohol, Beta-Sitosterol, Butyrospermum Parkii (Shea Butter), Euphorbia Cerifera (Candelilla) Wax) (LCW)
- Crosilk^{®} Liquid: ca. 27- 31% Festkörper (INCl-Bezeichnung: Aqua, Silk Amino Acids) (Croda)
- Cutina^{®} HR: gehärtetes Rizinusöl (INCl-Bezeichnung: Hydrogenated Castor Oil) (Cognis)
- Emulgade^{®} 1000NI: (INCI-Bezeichnung: Cetearyl Alcohol, Ceteareth-20) (Cognis)
- Lanette^{®} 14: Myristylalkohol (INCl-Bezeichnung: Myristyl Alcohol) (Cognis)
- Lowenol^{®} C-279: C₁₆₋₁₈-Fettalkohol mit ca. 2-EO-Einheiten (INCI- Bezeichnung: Ceteareth-2) (Lowenstein)
- Merquat^{®} 100: Poly(dimethyldiallylammoniumchlorid) (ca. 40% Festkörper; INCl-Bezeichnung: Polyquaternium-6) (Ondeo Nalco)
- Merquat^{®} Plus 3330: Polymeres quaternäres Ammoniumsalz auf Basis von Acrylsäure, Dimethyldiallylammoniumchlorid und Acrylamid (INCl-Bezeichnung: Polyquaternium-39) (Nalco)
- Polawax^{®} GP 200: (INCl-Bezeichnung: Cetearyl Alcohol, Peg-20 Stearate) (Croda)
- Violet 1,4 D: 1,4-Bis[(2'-hydroxyethyl)-amino]-2-nitrobenzol

## Patentansprüche

1. Zweikammertube, enthaltend in einer ersten Kammer eine Zubereitung (A), enthaltend mindestens einen direktziehenden Farbstoff und/oder mindestens eine Vorstufe eines naturanalogen Farbstoffs, ausgewählt aus den Indol- und/oder den Indolinderivaten, und in einer zweiten Kammer eine Zubereitung (B), enthaltend mindestens einen Pflegestoff, **dadurch gekennzeichnet, dass** sie eine innere und eine äußere Kammer aufweist, die beide in einem gemeinsamen Kopfbereich (Austrittsbereich) enden.

2. Zweikammertube nach Anspruch 1, **dadurch gekennzeichnet, dass** die Zubereitung (B) als Pflegestoff mindestens ein kationisches Tensid enthält.

3. Zweikammertube nach einem der Ansprüche 1 oder 2, **dadurch gekennzeichnet, dass** die Zubereitung (B) als Pflegestoff mindestens ein pflegendes Polymer enthält.

4. Zweikammertube nach einem der Ansprüche 1 bis 3, **dadurch gekennzeichnet, dass** die Zubereitung (B) als Pflegestoff mindestens einen UV-Filter enthält.

5. Zweikammertube nach einem der Ansprüche 1 bis 4, **dadurch gekennzeichnet, dass** die Zubereitung (B) als Pflegestoff mindestens ein Vitamin, ein Provitamin, eine Vitaminvorstufe und/oder eines derer Derivate enthält.

6. Zweikammertube nach einem der Ansprüche 1 bis 5, **dadurch gekennzeichnet, dass** die Zubereitung (B) als Pflegestoff mindestens einen Pflanzenextrakt enthält.

7. Zweikammertube nach einem der Ansprüche 1 bis 6, **dadurch gekennzeichnet, dass** die Zubereitung (B) als Pflegestoff mindestens ein Proteinhydrolysat und/oder eines seiner Derivate enthält.

8. Zweikammertube nach einem der Ansprüche 1 bis 7, **dadurch gekennzeichnet, dass** die Zubereitung (B) als Pflegestoff mindestens eine Verbindung, ausgewählt aus Ectoin oder Ectoinderivaten, Allantoin, Taurin und Bisabolol, enthält.

9. Zweikammertube nach einem der Ansprüche 1 bis 8, **dadurch gekennzeichnet, dass** die Zubereitung (B) als Pflegestoff mindestens ein Mono- bzw. Oligosaccharid enthält.

10. Zweikammertube nach einem der Ansprüche 1 bis 9, **dadurch gekennzeichnet, dass** die Zubereitung (B) als Pflegestoff mindestens ein Silikonöl und/oder ein Silikongum enthält.

11. Zweikammertube nach einem der Ansprüche 1 bis 10, **dadurch gekennzeichnet, dass** die Zubereitung (B) als Pflegestoff mindestens einen Ölkörper enthält.

12. Zweikammertube nach einem der Ansprüche 1 bis. 11, **dadurch gekennzeichnet, dass** die Zubereitungen in einem Volumenverhältnis von (A):(B) entsprechend 1:2 bis 5-1 aus der Tube austreten.

13. Zweikammertube nach einem der Ansprüche 1 bis 12, **dadurch gekennzeichnet, dass** die eine Zubereitung als Streifen in dem aus der anderen Komponenten gebildeten Austrittsstrang enthalten ist.

14. Verfahren zur Tönung von keratinischen Fasern, insbesondere menschlichen Haaren, **dadurch gekennzeichnet, dass** ein Zweikomponentenmittel aus einer Zweikammertube nach einem der Ansprüche 1 bis 13 herausgedrückt wird, die Anwendungszubereitung auf die Fasern aufgebracht wird und nach einer Einwirkungszeit wieder abgespült wird.

## Claims

1. A two-chamber tube, comprising in a first chamber a preparation (A) comprising at least one substantive dye and/or at least one precursor of a nature-analogous dye, selected from the derivatives of indole and/or of indoline, and in a second chamber a preparation (B), comprising at least one conditioning substance, **characterised in that** said tube possesses an inner and an outer chamber, both of which end in a common top region (outlet region).

2. The two-chamber tube according to Claim 1, **characterised in that** the preparation (B) comprises at least one cationic surfactant as the conditioning substance.

3. The two-chamber tube according to one of Claims 1 or 2, **characterised in that** the preparation (B) comprises at least one conditioning polymer as the conditioning substance.

4. The two-chamber tube according to one of Claims 1 to 3, **characterised in that** the preparation (B) comprises at least one UV filter as the conditioning substance.

5. The two-chamber tube according to one of Claims 1 to 4, **characterised in that** the preparation (B) comprises at least one vitamin, a provitamin, a vitamin precursor and/or one of their derivatives as the conditioning substance.

6. The two-chamber tube according to one of Claims 1 to 5, **characterised in that** the preparation (B) comprises at least one plant extract as the conditioning substance.

7. The two-chamber tube according to one of Claims 1 to 6, **characterised in that** the preparation (B) comprises at least one protein hydrolysate and/or one of its derivatives as the conditioning substance.

8. The two-chamber tube according to one of Claims 1 to 7, **characterised in that** the preparation (B) comprises at least one compound, selected from ectoin or ectoin derivatives, allantoin, taurine and bisabolol, as the conditioning substance.

9. The two-chamber tube according to one of Claims 1 to 8, **characterised in that** the preparation (B) comprises at least one mono- or oligosaccharide as the conditioning substance.

10. The two-chamber tube according to one of Claims 1 to 9, **characterised in that** the preparation (B) comprises at least one silicone oil and/or a silicone gum as the conditioning substance.

11. The two-chamber tube according to one of Claims 1 to 10, **characterised in that** the preparation (B) comprises at least one oily substance as the conditioning substance.

12. The two-chamber tube according to one of Claims 1 to 11, **characterised in that** the preparations emerge from the tube in a volumetric ratio of (A): (B) corresponding to 1: 2 to 5:1.

13. The two-chamber tube according to one of Claims 1 to 12, **characterised in that** the one preparation is comprised as stripes in the emerging strand constituted by the other component.

14. A method for tinting keratinic fibres, in particular human hair, **characterised in that** a two-component agent is squeezed out of a tube according to one of Claims 1 to 13, the application preparation is deposited onto the fibres, and after a contact time is rinsed off again.

## Revendications

1. Tube à deux chambres, contenant, dans une première chambre, une préparation (A) contenant au moins un colorant direct et/ou au moins un précurseur d'un colorant analogue à ceux que l'on trouve dans la nature, choisi parmi les dérivés d'indole et/ou les dérivés d'indoline, et, dans une deuxième chambre, une préparation (B) contenant au moins une substance reconstituante, **caractérisé en ce qu'**il présente une chambre interne et une chambre externe qui débouchent toutes deux dans une zone de tête commune (zone de sortie).

2. Tube à deux chambres, selon la revendication 1, **caractérisé en ce que** la préparation (B) contient, à titre de substance reconstituante, au moins un agent tensioactif cationique.

3. Tube à deux chambres, selon la revendication 1 ou 2, **caractérisé en ce que** la préparation (B) contient, à titre de substance reconstituante, au moins un polymère reconstituant.

4. Tube à deux chambres, selon l'une quelconque des revendications 1 à 3, **caractérisé en ce que** la préparation (B) contient, à titre de substance reconstituante, au moins un filtre UV.

5. Tube à deux chambres, selon l'une quelconque des revendications 1 à 4, **caractérisé en ce que** la préparation (B) contient, à titre de substance reconstituante, au moins une vitamine, une provitamine, un précurseur de vitamine et/ou un de leurs dérivés.

6. Tube à deux chambres, selon l'une quelconque des revendications 1 à 5, **caractérisé en ce que** la préparation (B) contient, à titre de substance reconstituante, au moins un extrait de plante.

7. Tube à deux chambres, selon l'une quelconque des revendications 1 à 6, **caractérisé en ce que** la préparation (B) contient, à titre de substance reconstituante, au moins un hydrolysat protéinique et/ou un de ses dérivés.

8. Tube à deux chambres, selon l'une quelconque des revendications 1 à 7, **caractérisé en ce que** la préparation (B) contient, à titre de substance reconstituante, au moins un composé choisi parmi l'ectoïne ou des dérivés de l'ectoine, l'allantoïne, la taurine et le bisabolol.

9. Tube à deux chambres, selon l'une quelconque des revendications 1 à 8, **caractérisé en ce que** la préparation (B) contient, à titre de substance reconstituante, au moins un monosaccharide, respectivement un oligosaccharide.

10. Tube à deux chambres, selon l'une quelconque des revendications 1 à 9, **caractérisé en ce que** la préparation (B) contient, à titre de substance reconstituante, au moins une huile de silicone et/ou une gomme de silicone.

11. Tube à deux chambres, selon l'une quelconque des revendications 1 à 10, **caractérisé en ce que** la préparation (B) contient, à titre de substance reconstituante, au moins un composé huileux.

12. Tube à deux chambres, selon l'une quelconque des revendications 1 à 11, **caractérisé en ce que** les préparations sortent du tube dans un rapport en volume de (A) à (B) s'élevant de 1:2 à 5:1.

13. Tube à deux chambres, selon l'une quelconque des revendications 1 à 12, **caractérisé en ce que** la première préparation est contenue sous la forme de bandes dans le boudin de sortie formé par l'autre composant.

14. Procédé pour le nuançage de fibres kératiniques, en particulier de cheveux humains, **caractérisé en ce qu'**on fait sortir par pression un agent à deux composants hors d'un tube à deux chambres selon l'une quelconque des revendications 1 à 13, on applique la préparation d'utilisation sur les fibres et on la fait disparaître par rinçage après l'avoir laissé agir pendant un certain temps.
